(19) Europäisches Patentamt
European Patent Office
Office européen des brevets

(11) EP 4 751 634 A1

(12) EUROPEAN PATENT APPLICATION

(43) Date of publication:
03.06.2026 Bulletin 2026/23

(21) Application number: 24215740.2

(22) Date of filing: 27.11.2024

(51) International Patent Classification (IPC):
A61B 1/00 (2006.01)    A61B 1/005 (2006.01)
A61B 34/20 (2016.01)    A61B 34/30 (2016.01)
A61B 90/00 (2016.01)

(52) Cooperative Patent Classification (CPC):
A61B 34/30; A61B 1/00006; A61B 1/009;
A61B 2034/2046; A61B 2034/301; A61B 2090/064;
A61B 2090/376

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AE Eindhoven (NL)

(72) Inventors:
• FOTOUHI, Javad
Eindhoven (NL)

• PATRICIU, Alexandru
Eindhoven (NL)
• BALICKI, Marcin Arkadiusz
Eindhoven (NL)
• KYNE, Sean
Eindhoven (NL)
• SINHA, Ayushi
Eindhoven (NL)

(74) Representative: Philips Intellectual Property &
Standards
High Tech Campus 52
5656 AG Eindhoven (NL)

(54) **MEDICAL DEVICE MONITORING**

(57) The present disclosure provides concepts for monitoring a medical instrument comprising an elongate device and an actuator configured to control movement of the elongate device. In particular, device data indicating a shape and/or position of at least a portion of the elongate device, and actuator data indicating a target shape and/or position of at least the portion of the elongate device, are leveraged to monitor tension of the elongate device. The device data and actuator data are processed with a tension prediction algorithm to generate a predicted tension value describing a degree of resistance against movement experienced by the elongate device. This allows for real-time automatic monitoring of tension in the elongate device during medical procedures, enabling early detection of potentially dangerous situations and improving patient safety.

FIG. 2

**Description**

FIELD OF INVENTION

The present invention relates to systems and methods for monitoring tension in elongate medical devices, and in particular, to predicting and categorizing tension within robotically navigated elongate medical devices, such as flexible endovascular devices.

BACKGROUND

In minimally invasive endovascular procedures, precise control and manipulation of elongate medical devices such as catheters, guidewires, and stents are crucial for successful outcomes. These flexible devices are navigated through complex vascular structures to reach target sites for diagnosis or treatment. As these devices are advanced, tension can build up within the device body due to friction, anatomical constraints, and device properties. Proper tension management is essential to maintain control and prevent unexpected device movements that could potentially damage delicate vascular tissues.

Traditionally, interventionalists rely on haptic feedback and/or visual cues from fluoroscopic imaging to assess and manage tension in endovascular devices. They continuously monitor device progress and adjust tension as needed to ensure appropriate transmission of torque and translational forces to the device tip. However, this approach is highly dependent on the operator's experience and subjective interpretation of tactile and visual information. In robotic-assisted procedures, where direct haptic feedback is absent, tension management becomes even more challenging.

Current systems lack the ability to objectively predict, quantify, and categorize tension within robotically navigated flexible endovascular devices. This limitation can lead to suboptimal device control, increased procedure times, and potential safety risks. Therefore, there is a present need for automatic tension monitoring and prediction techniques that are accurate and reliable.

SUMMARY OF INVENTION

The invention is defined by the claims.

According to an aspect of the present disclosure, a system for monitoring a medical instrument is provided. The system includes an elongate device, and an actuator configured to control movement of the elongate device. The system comprises an input interface configured to obtain device data indicating a shape and/or position of at least a portion of the elongate device, and to obtain actuator data indicating a target shape and/or position of at least the portion of the elongate device. The system further comprises a processor configured to process, with a tension prediction algorithm, the device data and the actuator data to generate a predicted tension value describing a degree of resistance against movement experienced by the elongate device.

Proposed concepts thus aim to provide schemes, solutions, concepts, designs, methods, and systems pertaining to monitoring and predicting tension in elongate medical devices during minimally invasive endovascular procedures. In particular, embodiments aim to provide improved safety and control in robotic-assisted interventions where direct haptic feedback is absent. This system allows for real-time monitoring of tension in the elongate device during medical procedures, enabling early detection of potentially dangerous situations and improving patient safety.

More particularly, there is provided as system for monitoring a medical instrument comprising an elongate device (e.g., catheters, guidewires, and stents) and an actuator configured to control movement of the elongate device. In particular, device data indicating a shape and/or position of at least a portion of the elongate device, and actuator data indicating a target shape and/or position of at least the portion of the elongate device, are leveraged to monitor tension of the elongate device. Indeed, it has been realized that a difference between device data indicating the actual shape and/or position of the device and actuator data indicating the target/intended shape and/or position of the device correlates with the degree of tension of the elongate device.

The device data and actuator data are processed with a tension prediction algorithm to generate a predicted tension value describing a degree of resistance against movement experienced by the elongate device. This allows for real-time automatic monitoring of tension in the elongate device during medical procedures, enabling early detection of potentially dangerous situations and improving patient safety.

In other words, a core aspect of the invention is the use of device data describing the actual shape and position of the device (e.g., image data or sensor data) and comparing that to actuator data describing the expected shape and/or position of the device (e.g., robotic input data). From a difference between these, the tension can be predicted. To this end, a tension prediction algorithm is employed that can map received device data and actuator data to predicted tension values.

Thus, the invention provides a novel means for monitoring the degree of tension (and conversely the slack) of the elongate device by assessing a difference between an actual and expected/target shape, position and/or movement of the device. This is of key importance to ensure appropriate transmission of torque and translational forces to the device tip by the actuator.

In some embodiments, the processor may be further configured to categorize the predicted tension value into one of a plurality of classes, wherein the plurality of classes comprise at least one class associated with tension values in a safe range, and at least one class associated with tension values in a dangerous range.

The system may therefore categorize predicted tension values into different classes, such as safe and dangerous ranges. This categorization provides clear, easily interpretable information to guide decision-making during procedures, and using which appropriate automated action may be taken to avoid potentially unsafe situations. Of course, there may be many classes indicating a wide range of degrees of tension using which a more nuanced action may be taken.

The tension prediction algorithm may be a machine-learning based algorithm. Utilizing a machine-learning based algorithm may allow the system to infer the tension in scenarios unseen in training data, and thus provides an accurate and reliable means for predicting the tension in a wide-range of real-world situations. This may also allow the system to continuously improve its prediction accuracy over time as it processes more data.

The machine-learning based algorithm may be trained using a sequence of device data and corresponding actuator data, and associated tension values. Training the algorithm with real-world data ensures that it can accurately predict tension values across a wide range of medical procedures and device configurations.

In some embodiments, the processor may be further configured to process, with the tension prediction algorithm, the device data and the actuator data to generate a predicted future tension value describing an expected change of the degree of resistance against movement experienced by the elongate device.

Predicting future tension values allows for proactive adjustments to be made, potentially preventing dangerous situations before they occur. Indeed, the previous build-up (or release of) tension provides an indication for the future change of tension and thus may be useful to prevent tension of the elongate device changing into an unsafe range.

The device data may indicate a shape and/or position of the portion of the elongate device over time, and the actuator data may indicate the target shape and/or position the portion of the elongate device over time.

Analyzing data over time provides a more comprehensive understanding of the device's behavior and allows for more accurate tension predictions. Indeed, a full appreciation of the current state of tension of the elongate device, and the current rate of change of state of tension of the elongate device, may only be achieved if the rate of change of difference between the target/intended and actual shape, position and motion of the elongate device is known.

More specifically, the device data may comprise image data of at least the portion of the elongate device. Using image data allows for direct visualization of the device's actual shape and position, providing more accurate input for the tension prediction algorithm.

In particular, the image data may comprise a fluoroscopy image of at least the portion of the elongate device. Fluoroscopy images provide real-time, high-quality visualization of the device within the patient's body, enabling precise tracking of the device's movement and shape. Such images are usually taken during endovascular procedures, and therefore this data may be readily available.

Furthermore, the actuator data may comprise input data describing one or more prompted actions of the actuator to control movement of the elongate device.

Including actuator input data allows the system to derive the intended/target shape of the elongate device as triggered by the actuator. Thus, correlation between intended device movements with actual device behavior can be determined, improving the accuracy of tension predictions.

In some embodiments, the predicted tension value may be further based on one or more characteristics of the elongate device.

Considering device characteristics allows the system to account for variations in device properties, such as stiffness or flexibility which may vary along the length of the elongate device, leading to more accurate tension predictions across different types of devices. Furthermore, different degrees of tension may be expected for different elongate devices.

In further embodiments, the system may also comprise an output interface configured, based on the predicted tension value, to: control the actuator to adjust the shape and/or position of the elongate device; control the actuator to cease movement of the elongate device; provide a notification to the user indicating the predicted tension value; provide a recommendation to the user indicating a recommended alternative input; and/or control a capture rate at which the device data is obtained.

These output options provide a range of responses to predicted tension values, from automated adjustments to user notifications, enhancing the system's ability to prevent dangerous situations and optimize device control.

According to another aspect of the present disclosure, a medical device arrangement is provided. The medical device arrangement comprises an elongate device, an actuator configured to control movement of the elongate device, and the system for monitoring a medical instrument of any embodiment described herein.

In some embodiments, the actuator may be controlled based, at least in part, on the predicted tension value.

Controlling the actuator based on predicted tension values allows for automated adjustments to device movement, potentially preventing dangerous situations and optimizing device performance.

According to a further aspect of the present disclosure, a method for monitoring a medical instrument is provided. The method comprises obtaining device data indicating a shape and/or position of at least a portion of an elongate device, obtaining actuator data indicating a target shape and/or position of at least the portion of the elongate device, and processing, with a tension prediction algorithm, the device data and the actuator data to generate a predicted tension value describing a degree of resistance against movement experienced by the elongate device.

According to additional aspects of the present disclosure, a computer program comprising computer program code means adapted, when said computer program is run on a computer, to implement the method described above is provided.

These and other aspects of the invention will be apparent from and elucidated with reference to the embodiment(s) described hereinafter.

BRIEF DESCRIPTION OF FIGURES

For a better understanding of the invention, and to show more clearly how it may be carried into effect, reference will now be made, by way of example only, to the accompanying drawings, in which:

FIG. 1 illustrates a simplified schematic view of an elongate medical device progressing through example vasculature, according to an example.
FIG. 2 depicts a block diagram of a medical instrument monitoring system, in accordance with example embodiments.
FIG. 3 shows a flowchart for a method of monitoring tension in a medical instrument, according to an embodiment; and
FIG. 4 is a simplified block diagram of a computer within which one or more parts of an embodiment may be employed.

DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for monitoring a medical instrument comprising an elongate device and an actuator configured to control movement of the elongate device. In particular, device data indicating a shape and/or position of at least a portion of the elongate device, and actuator data indicating a target shape and/or position of at least the portion of the elongate device, are leveraged to monitor tension of the elongate device. The device data and actuator data are processed with a tension prediction algorithm to generate a predicted tension value describing a degree of resistance against movement experienced by the elongate device. This allows for real-time automatic monitoring of tension in the elongate device during medical procedures, enabling early detection of potentially dangerous situations and improving patient safety.

The invention thus provides a system and method for predicting, identifying, and categorizing tension within robotically navigated flexible endovascular devices (e.g., catheters, guidewires, and stents) by correlating patterns within device data (e.g., acquired from medical imaging data, sensor data) and actuator data (e.g., actuator input data, output data, etc.). This approach enables real-time monitoring of device tension without requiring specialized hardware or modifications to existing medical instruments.

To be clear, in minimally invasive endovascular procedures, it is essential to continuously evaluate the tension (and conversely the slack) in the elongate device and adjust it as required. Tension management is particularly important when navigating delicate structures, such as brain aneurysms, where built-up resistance due to poor tension management can cause the device to damage the surrounding tissue.

Tension is the buildup of energy in the device body when translational and/or rotational input provided at the proximal end of the elongate device (typically outside patient body and manipulated by user/robot) does not result in expected motion at the distal end of the device (device tip inside the patient body). Accordingly, greater tension reflects the increased resistance against movement experienced by the elongate device. With a very high resistance against movement, the elongate device may not move (or may move very little) with input movement. This reflects a build-up of tension of the elongate device. In some cases, build-up of tension may cause the device body to loop, especially in larger vessels, causing the device to buckle. This can result in parts of the device body pushing up against vessel walls increasing the risk of vessel perforation. Further, a sudden release of the tension may cause the elongate device to change position suddenly, releasing energy which may cause damage to surrounding tissue. Equally, when there is no resistance to movement, an input movement at one end of the device may equate to equivalent movement of the distal end of the device - reflecting an appropriate amount of built-up tension in the device to enable such one-to-one movement. An elongate device in this context could mean a single elongate device or a stack or system of elongate devices (e.g., co-axial system of devices such as a catheter with a guidewire within it).

Tension built up in the device body can give the device spring-like properties such that with enough energy built up, the energy can release propelling the device tip forward. Further, it is crucial to reduce the tension before entering an aneurysm to achieve proper control of rotation and translation and avoid perforation. If the tension is not removed sufficiently, the torque will not transmit appropriately to the tip of the elongate device - meaning that the device will not adopt the desired shape.

Commonly, a build-up of tension is detected by the user by haptic feedback. That is, if the user experiences some resistance in applying an input, this may imply a build-up of tension in the elongate device. It is important to first identify when tension build-up is present, and to then determine when it should be removed. However, this is an unreliable mean for detecting tension, with the haptic feedback dependent on many other factors such as experience, accompanied visual feedback, etc. Furthermore, while this is possible for a human operator, applying the same strategy to a robot that is being controlled via joystick or other controllers or that is automatically navigating and has no notion of haptic response is not attainable. Indeed, it is inherently difficult to automatically detect and analyze such changes, and therefore this feedback is not practical for implementation in automated systems.

Thus, the proposed invention predicts the amount of tension and may categorize it before high and undesired tension occurs. Early prediction of tension can contribute to increased procedure safety. This is because the physicians (or the autonomous system) are informed earlier and can reduce tension before it causes undesirable device movements. This is achieved by comparison of the intended/target device shape, location/position and motion (as induced by an actuator), and the actual device shape, location/position and motion (as intended by an actuator). A disparity between this data may indicate the presence and build-up of tension in the elongate device.

A key aspect of the invention is the use of a tension prediction algorithm, which may be implemented as a machine learning model. This algorithm processes device data (such as fluoroscopic images showing device shape and position) along with actuator data (indicating target device movements) to generate predicted tension values. These predictions allow for early detection of potentially dangerous situations, enabling proactive adjustments to device manipulation.

The system may categorize predicted tension values into different classes, such as safe and dangerous ranges. This categorization provides clear, easily interpretable information to guide decision-making during procedures. Furthermore, the system may generate predictions of future tension values, allowing for anticipatory actions to prevent unsafe conditions before they occur.

By integrating this tension monitoring capability with robotic control systems, the invention enables automated responses to predicted tension values. These responses may include adjusting device movement, ceasing movement, or recommending alternative actions to the operator. This integration of predictive monitoring with robotic control has the potential to significantly enhance the safety and efficacy of minimally invasive endovascular procedures.

Overall, embodiments of the invention predict if and when further movement of the elongate device (e.g., affected by the actuator) will result in high tension. In other words, embodiments predict high tension before high tension occurs. It is then possible to inform the user/system that unordinary build-up of tension has and/or will occur, and thus may recommend alternative inputs to reduce the likelihood of further build-up of tension. Predicting high buildup of tension before there is a large amount of tension (or even before buckling) is novel and particularly useful for instances where the actuator is controlled autonomously. Thus, when it is indicated that there is a high degree of tension in the elongate device, the elongate device may be pulled-back, for example, to relax the tension.

To this end, actuator data, such as the inputs to the actuator by the user or generated by the autonomous controller (e.g., push/pull/twist commands to actuate each device, velocity, etc.), system accelerations, system forces, device stack characteristics (e.g., type of each device loaded, length of the elongate device, and/or stiffness of each elongated device, etc.), and device data, such as a sequence of medical imaging data and insights extracted from the imaging data (e.g., device tip position, device shape, anatomical landmarks, roadmaps, etc.) or pressure data from sensors along the elongate device are combined. Correlations between the device data, actuator data and tension in the elongate device can be estimated/approximated, and thus a predicted tension is generated responsive to the device data and actuator data.

Put another way, the correlations and common patterns between device data (indicating the actual shape, position and/or movement of the device) and actuator data (indicating an intended shape, position and/or movement of the device as affected by the actuator) are used to distinguish different levels of tension in the elongate device. In one example, a ratio, r, of movement on the proximal end of the device to the movement at the distal tip is predicted from the actuator data and device data:

$$r = \frac{\frac{d}{dt}p}{\frac{d}{dt}a}, \qquad \frac{d}{dt}a \neq 0, \qquad\qquad [1]$$

where $p$ and $a$ denote device data and actuator data, respectively, acquired over some time interval (e.g., 5 seconds), and $\frac{d}{dt}$ represents the gradient or derivative with respect to time, t, which provides the change in the device data and actuator data over some time interval. The ratio, r, is calculated only when change in actuator data is non-zero because when the actuator is not providing any input, there is no tension being built-up in the elongate device.

From the deviation in this ratio from an expected 1:1 ratio (which would arise if the elongate device experienced no resistance to movement), tension in the elongate device can be predicted.

For example, if a user (or an automated controller) generates commands to translate the device with a significant expected displacement of the device tip (which is reflected in the actuator data), but the device tip does not move as expected according to the device data, then it may be concluded that there is a high degree of resistance to movement, and therefore the likelihood of build-up of tension in the system is high. It is important to note that the extent of displacement in the device tip serves as a crucial indicator. Additionally, the shape of the device also functions as another key indicator of tension within the elongate device.

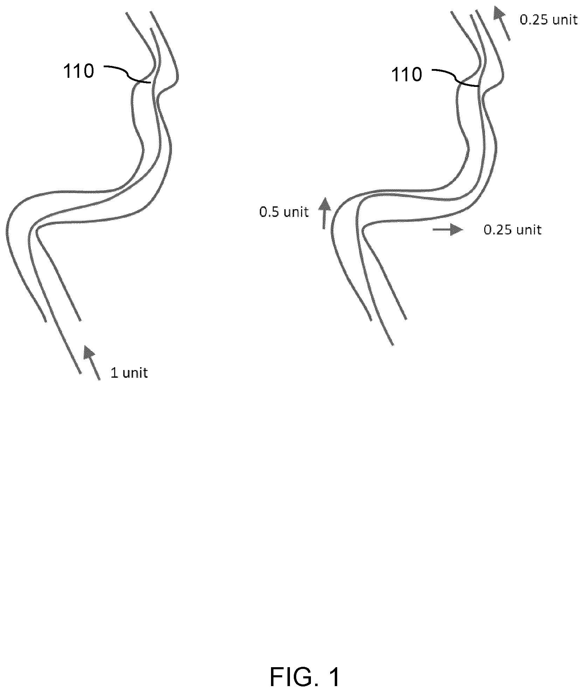

Referring to FIG. 1, the Figure illustrates the concept of tension in an elongate medical device, such as a catheter, guidewire, or stent, by showing the relationship between input movement at the proximal end and resulting movements along the device's length.

The elongate device 110 is controlled by an actuator, which may be part of a robotic system. The actuator is configured to control the movement of the elongate device 110 based on input data, which describes one or more prompted actions of the actuator to control the movement of the elongate device 110.

As shown in the left Figure, the proximate end of the elongate device 110 is moved by the actuator 1 unit (e.g., 1mm, 1cm, etc.). Accordingly, it may be expected that the whole elongate device is moved 1 unit in the direction applied by the actuator, resulting in the distal end of the elongate device 110 also moving 1 unit. This is reflected in the actuator data of the present invention.

However, as seen in the right Figure, due to the shape and size of the vasculature, this presumption is not true. In this case, the shape of the elongate device deforms due to contact with walls of the vasculature. Furthermore, the movement of the elongate device is not solely in the direction of applied movement at the proximate end. As a result, the distal end only moves 0.25 units in the direction of the applied movement. Thus, the change in shape of the elongate device absorbs the remaining 0.75 units of the applied movement. This actual movement and change of shape are reflected in the device data (e.g., sensor data or image data). In an example, the change in shape or curvature, c, of the elongate device over time may be predicted from the device data using the following:

$$c = \sqrt{p_x''^2 + p_y''^2}, \qquad\qquad [2]$$

where $p''$ denotes the second derivative of the x and y components describing the coordinates of the shape of the device and c is the curvature. Then, $\frac{d}{dt}c$, which is the derivative with respect to time over some time interval, describes the change in curvature of the device over that time interval.

Using both the movement at the distal tip and the change in curvature of the device to estimate tension in the device is important because tension affects the full device and using only one source of information may not provide sufficient data.

For example, by only looking at the distal tip of the device, instances where the distal tip is not moving and instances where the distal tip is moving into or out of the image plane may be indistinguishable. This is because X-ray imaging is a 2D projection imaging modality and a distal tip moving into or out of the image plane may be occluded by the shape of the device, giving the appearance of a non-moving or static distal tip. In this case, the change in curvature of the device may provide insight into whether the distal tip is truly static or occluded. That is, if the curvature of the device does not significantly change, this can indicate that the distal tip may be moving into or out of the imaging plane, and the estimated ratio, r, may be weighted down in order to denote continue low tension.

In contrast, if lack of movement at the distal tip is accompanied by change in curvature of the device, then the ratio, r, may be displayed as is, and if lack of movement at the distal tip is accompanied by significant change in curvature of the device, then the ratio, r, may be weighted up in order to denote a large increase in tension. Similarly, by only looking at the change in curvature, it may not be clear whether the change is caused by the changing curvature of the vasculature or because the movement affected by the actuator is not being translated to movement at the distal tip and is instead being absorbed by the shape of the elongate device.

The difference between the expected or target movement affected by the actuator, and the actual movement of the elongate device is related to the degree and type of resistance against movement experienced by the elongate device (i.e., the tension experienced by the elongate device). This degree of resistance/tension value may vary across the length of the elongate device depending on positioning and local elongate device stiffness. The means for translating the difference is provided by the tension prediction algorithm, which is trained to receive device and actuator data, and output a predicted tension value.

For example, the tension prediction algorithm may be a machine learning algorithm, such as a neural network system, that is trained using two main data sources: (i) a single medical image or a sequence of medical images (e.g., fluoroscopy images, DSA, 3D CBCT, device shape segmentation, device position data, etc.), and (ii) data indicating the state of the actuator (user inputs, system generated commands, encoder data, joint positions, joint velocities, joint accelerations, forces, event logs, timestamps, etc.). Since the combinations of various types of information that indicate the amount of tension in the elongate device including the ratio, r, change in curvature, $\frac{d}{dt}c$, etc. can be complex, training a machine learning algorithm using large sets of prior data can result in more accurate tension predictions than hand-tuned use of this information, as described above. In this case, the tension prediction algorithm learns how to map various information including movement at the distal tip (e.g., as observed in pixel-space from image data) relative to movement affected by the actuator (e.g., as observed in millimeter-space from robot data), change in device curvature, etc. to a tension value. The predicted tension may be a continuous value determined from a regression based neural network architecture that ingests input information and directly outputs a tension value:

Input: $p = \{..., p_{t-2}, p_{t-1}, p_t\}$, $a = \{..., a_{t-2}, a_{t-1}, a_t\}$, $c = \{..., c_{t-2}, c_{t-1}, c_t\} \rightarrow$ Output: $s$, tension value.

Alternatively, predicted tension may be classified into one of several classes by training a classification based neural network that ingests input information and directly outputs one class (e.g., one of low, medium, or high):

Input: $p = \{..., p_{t-2}, p_{t-1}, p_t\}$, $a = \{..., a_{t-2}, a_{t-1}, a_t\}$, $c = \{..., c_{t-2}, c_{t-1}, c_t\} \rightarrow$ Output: $s_{class}$, a tension class (e.g., [1, 0, 0] or [0, 0, 1], where the positions in the array represent low, medium, and high tension and [1, 0, 0] classifies tension as low, while [0, 0, 1] classifies tension as high).

In both examples described above, training the neural network will require a ground truth tension value or tension class in order to compute a loss function comparing the predicted tension value or tension class with the ground truth tension value or tension class. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized over several iterations, and training is terminated when the value of the loss function satisfies one or multiple stopping criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers". These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the loss, or difference between the predicted output data and the ground truth data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

If ground truth data is not available for all training data, the neural network may be trained using self-supervised learning and/or contrastive learning using a neural network architecture that ingests input information and outputs the same input information from a reduced dimension latent space:

Input: $p = \{..., p_{t-2}, p_{t-1}, p_t\}$, $a = \{..., a_{t-2}, a_{t-1}, a_t\}$, $c = \{..., c_{t-2}, c_{t-1}, c_t\} \rightarrow e \rightarrow$ Output: $\hat{p} = \{..., \hat{p}_{t-2}, \hat{p}_{t-1}, \hat{p}_t\}$, $\hat{a} = \{..., \hat{a}_{t-2}, \hat{a}_{t-1}, \hat{a}_t\}$, $\hat{c} = \{..., \hat{c}_{t-2}, \hat{c}_{t-1}, \hat{c}_t\}$, where $e$ is the latent encoding in the reduced dimension, and $\hat{p}$, $\hat{a}$, and $\hat{c}$ are the output or estimated device data, actuator data, and curvature data, respectively.

The loss function in this case compares the input and output, and the neural network learns a representation of input information in latent space. Contrastive learning further compares latent encodings from inputs when ground truth is available such that the distance between encodings from inputs associated with the same tension class is minimized and distance between encodings from inputs associated with different tension classes is maximized, allowing the neural network to learn a separable representation of input information in latent space. These representations can be clustered (e.g., into low, medium, and high-tension clusters). The trained neural network, based on the learned representations, then clusters current tension into one of different levels of tension (e.g., low, medium, or high) depending on distance of the encoding of the current input from each of the various clusters (e.g., assign tension class of the closest cluster).

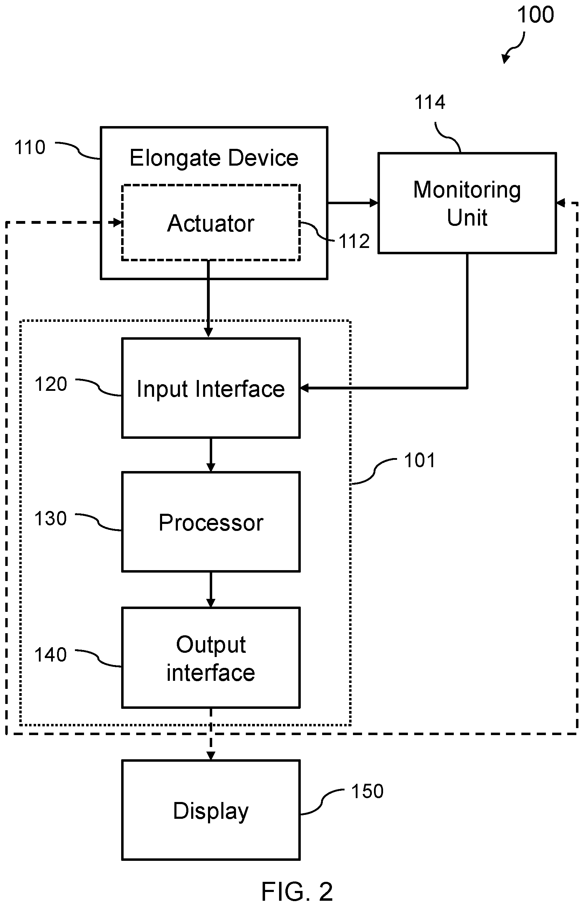

Referring to FIG. 2, there is depicted a medical device arrangement 100 comprising a medical instrument including an elongate device 110 and an actuator 112, and a system 101 for monitoring the medical instrument.

The medical instrument may be any device suitable for endovascular, endoscopic, or any other minimally invasive procedure that requires insertion into a body. The elongate device 110 is the part of the medical instrument that is inserted into the body and may be moved through the body in order to perform the procedure. For example, the elongate device 110 may be catheters, guidewires, stents, or any other component having an elongate body for entering into the body. In some cases, the elongate device 110 may comprise a coupled set of devices for insertion into the body at the same time. In this case, the coupled devices forming the elongate device 110 may be concentrically or co-axially arranged or wound together.

The actuator 112 is configured to control movement of the elongate device 110. That is, the actuator 112 is configured to affect/cause movement of the elongate device 110 within the body. To this end, the actuator 112 may apply various displacements and rotations to at least a proximate end of the elongate device 110 (i.e., an end of the elongate device 110 opposite the end that is intended to be furthest inside the body or any point along the portion of the device outside the patient body that the user or robot can grasp). In other embodiments, the actuator 112 may control movement of other parts of the elongate device 110 along the length of the elongate device 110, thus providing for finer control of the shape and position of the elongate device 110. The actuator 112 may be controlled automatically by a controller or may be controlled by a user providing inputs to the actuator 112.

The system 101 for monitoring the medical instrument comprises at least an input interface 120, and a processor 130, and may further comprise an output interface 140.

The input interface 120 is configured to obtain device data and actuator data.

The device data indicates the shape and/or position of at least a portion of the elongate device 110. That is, the device data describes the actual shape, position and movement/motion of the elongate device 110. This data may be obtained from various sources, such as medical imaging devices, sensors embedded in the elongate device 110, or other suitable sources. In the depicted embodiment, the device data is obtained from a monitoring unit 114, which measures the shape and/or position of at least a portion of the elongate device 110. To this end, the monitoring unit may be a medical imaging unit, or an interface for a plurality of sensors embedded in the elongate device 110.

In exemplary embodiments, the device data may comprise image data of at least the portion of the elongate device 110. Specifically, the image data may include fluoroscopy images of the elongate device 110.

The actuator data indicates the target shape and/or position of the same portion of the elongate device 110. That is, the actuator data describes the shape, position and movement/motion of the elongate device 110 should the elongate device 110 experience no resistance to movement. This data may be based on user inputs, pre-programmed instructions, or other suitable sources. Thus, the actuator data may be obtained directly from the actuator 112 itself. For example, the actuator data may be based on controls from the user, such as prompting the elongate device 110 to be twisted, rotated, or translated by the actuator 112. Alternatively, the actuator data may be based on encoded components of the robot including movement of robot arms, joints, rollers, etc.

Accordingly, the actuator data may describe the movements applied to a proximal end of the elongate device 110 by the actuator 112. From this described movement, the expected shape, position and/or movement of other parts of the elongate device 110 (e.g., the distal end/tip) may be derived.

To be clear, the device data and actuator data may indicate the respective actual and target shape and/or position of the elongate device 110 at a single time point. In further embodiments, the device data indicates a shape and/or position of the portion of the elongate device 110 over time, and the actuator data indicates the target shape and/or position the portion of the elongate device 110 over time. Thus, the change and rate of change of the actual and expected/target position, shape and motion of the elongate device 110 may be derivable from the device and actuator data.

The processor 130 receives the device data and the actuator data from the input interface 120. The processor 130 then processes the device data and the actuator data with a tension prediction algorithm. This algorithm generates a predicted tension value that describes the degree of resistance against movement experienced by the elongate device 110 based on the received device data and actuator data. In other words, a prediction as to the degree of resistance against movement experienced by the elongate device 110 is predicted, which correlates with the accumulation of energy and the buildup of tension in the elongate device 110.

The predicted tension value may be partially based on a ratio of the displacement of the device tip (e.g., derivable from the device data) and the displacement of the proximate tip or portion of the elongate device 110 (e.g., derivable from the actuator data).

The predicted tension value may be an absolute value reflecting a value of tension in the elongate device.

Alternatively, the predicted tension value may indicate a relative value of tension build up in the elongate device (e.g., high, medium, low tension). In some aspects, the processor 130 may further categorize the predicted tension value into one of several classes. These classes could include at least one class associated with tension values in a safe/low range, and at least one class associated with tension values in a dangerous/high range. Furthermore, these classes may include a more granular prediction of the tension value.

In some cases, the processor 130 is further (or alternatively) configured to process, with the tension prediction algorithm, the device data and the actuator data to generate a predicted future tension value describing an expected change of the degree of resistance against movement experienced by the elongate device 110. In other words, the processor 130 is configured to predict the buildup of tension in the elongate device, and thus predict whether the tension is likely to increase to a high/dangerous level. For example, a neural network may be trained for predict future tension using data available from past procedures:

Input: $p = \{..., p_{t-2}, p_{t-1}, p_t\}$, $a = \{..., a_{t-2}, a_{t-1}, a_t\}$, $c = \{..., c_{t-2}, c_{t-1}, c_t\} \rightarrow e \rightarrow$ Output: $\hat{p} = \{p_{t+1}, p_{t+2}, p_{t+3}, ...\}$, $\hat{a} = \{a_{t+1}, a_{t+2}, a_{t+3}, ...\}$, $\hat{c} = \{c_{t+1}, c_{t+2}, c_{t+3}, ... \}$, where $\hat{p}$, $\hat{a}$, and $\hat{c}$ are device data, actuator data, and curvature data, respectively, from a future time interval.

Since this future information is available from past procedures, the loss function in this case compares the output and the ground truth device data, actuator data, and curvature data from the future time interval. Due to this being a more difficult task for the neural network to learn, this may allow the network to learn more meaningful latent representations. Such a network also enables the computation of future parameters (e.g., future ratio, $\hat{r}$, from $\hat{p}$ and $\hat{a}$, and/or future change in curvature, $\frac{d}{dt}\hat{C}$, from $\hat{c}$), in addition to the cluster assignment (e.g., low, medium, high) for the current inputs.

Additionally, the predicted output can in turn be input into the neural network to obtain an encoding of the predicted future information along with its cluster assignment. If the cluster assignment of the current input is medium and that of the predicted future information is high, an early warning of a dangerous level of tension is made available to the user/robot.

In a combination of regular predictions and future prediction, a neural network may be trained to predict future device data and curvature data while learning to reproduce the input actuator data from the reduced dimension representations. This makes the future prediction task slightly easier by teaching the neural network to predict future device and curvature data assuming that the actuator data will remain the same, i.e., the user will continue to provide the current set of inputs to the robot or the robot, operating autonomously, will continue to provide the same inputs. If a future high tension is predicted, the user/robot will change the inputs provided to the robot (i.e., actuator data) such that a future high tension is avoided. This data, in turn, can be used to train the neural network to predict actuator data that will result in low or medium future tension.

In addition, the predicted tension value may be further based on one or more characteristics of the elongate device 110. That is, while the examples above only take into account device data, actuator data, and curvature data, the tension prediction algorithm may also take into account various additional aspects of the elongate device 110 when predicting the tension value. This may correspond to various device properties, such as stiffness (either overall, or the stiffness in different parts of the elongate device 110), joint position, vasculature, etc.

To reiterate, the tension prediction algorithm is configured to receive actuator data and device data, and to provide a tension value prediction based on the received actuator data and device data. To this end, the tension prediction algorithm may be a machine-learning based algorithm that is trained on training data to learn relationships between the actuator data, device data, and tension state of the elongate device 110. Nevertheless, the tension prediction algorithm is not restricted hereto, and may be a rules-based algorithm, for example.

The machine-learning based algorithm may be trained using a sequence of device data and corresponding actuator data, and associated tension values. These values may be generated from real-life scenarios or may be generated based on a simulation.

By way of example, in a data-driven and neural network-based implementation of the tension prediction algorithm, unsupervised training of the neural network can be used to train the tension prediction algorithm. This does not require large-scale labeling of data. To design this network, for instance, an encoder-decoder architecture can be used that learns the mapping between device data (e.g., fluoroscopy images of the elongate device 110) and actuator data (e.g., the actuator's 112 commands). The input and output of the neural network can simultaneously include both the device data and the actuator data.

Within the latent space of the neural network, the data sets corresponding to high tension will cluster separately from the data sets corresponding to low or medium tension. It should also be noted that the latent space can include more than two clusters and reveal multiple levels of tension in the elongate device (and potentially among different devices, where the tension prediction algorithm is configured to predict tension values for more than one elongate device/device type).

During inference, if the obtained device and actuator data input to the neural network corresponds to the latent space closer to the cluster corresponding to high tension, the tension prediction algorithm will output a predicted tension value corresponding to high tension in the elongate device 110.

In another example, the tension prediction algorithm may be trained in a supervised manner with pre-annotated data, for example acquired from previous interventions with the elongate device 110. The actuator data and device data may be acquired during the intervention and correspond to various labels describing the level of tension building up in the elongate device. The actuator data and device data may be from a single point during the intervention or may be a sequence of data points throughout the intervention.

Of course, a combination of supervised and unsupervised training of the tension prediction algorithm may be performed. Furthermore, alternative means of gathering device and actuator data and labelling the data for training will be readily appreciated.

To summarize, the tension prediction algorithm may be trained using a single device data (e.g., stand-alone images of the elongate device 110), or a sequence of device data (e.g., sequences of images). This device data is associated with actuator data describing the robotic state of the actuator (e.g., user inputs, system commands, encoder data, joint positions, joint velocities, joint accelerations, forces, logs, timestamps, etc.). The tension prediction algorithm learns the mapping from the device data to the actuator data (e.g., the image-space to actuator-space) and, based on the learned representations, will cluster different levels of tension separately.

Moving on, the output interface 140 is configured to perform various actions based on the predicted tension value generated by the processor 130.

In a first embodiment, the output interface 140 is configured, based on the predicted tension value, to control the actuator 112 to adjust the shape and/or position of the elongate device 110. For example, the actuator 112 may be controlled to remove tension by either pushing, pulling and/or twisting the elongate device 110 when high levels of tension are detected. This allows for proactive adjustments to be made, potentially preventing dangerous situations and complications in the medical procedure before they occur.

The output interface 140 may control the actuator 112 depending on the degree of tension. The actuator 112 may be controlled so as to reduce the level of tension until the predicted tension value is beneath a predetermined threshold (indicating an excess of tension). This predetermined threshold may be fixed, may be based on user preferences, or may be changed depending on the identified location of the elongate device 110 in the anatomy (e.g., the threshold is reduced if it is determined that the elongate device 110 is in a sensitive region).

Further, the output interface 140 may control the actuator 112 to stop/cease movement of the elongate device 110. That is, if a high buildup of tension is predicted, movement of the elongate device 110 may be prevented to avert an unsafe situation.

In some cases, the output interface 140 may provide a notification to the user indicating the predicted tension value. This notification can alert the user to the potential tension in the elongate device 110, allowing the user to take appropriate action to manage the tension. The notification may be provided through various means, such as visual alerts on the display 150, auditory alerts, or tactile alerts.

For example, the notification may inform the user of buildup of tension of the elongate device, so that they may alter future inputs to the actuator. The notification may indicate where the build up of tension is occurring, so that they may take appropriate action. Alternatively, or additionally, the notification may describe the movement of the elongate device 110 that is expected to lead to an unsafe build up of tension.

In certain embodiments, the output interface 140 may provide a recommendation to the user indicating a recommended alternative input to the actuator 112. This recommendation can guide the user to adjust the control of the elongate device 110 in a way that can reduce the tension. The recommendation may be based on the predicted tension value and other relevant factors, such as the characteristics of the elongate device 110 and the current state of the medical procedure.

In further embodiments, the recommendation may be a recommendation to use an alternative elongate device 110, as the current elongate device 100 cannot be safely used to navigate the structure without unsafe build up of tension.

Furthermore, the output interface 140 may control a capture rate at which the device data is obtained by the input interface 120. This may be achieved by changing parameters of the monitoring unit 114. By adjusting the capture rate based on the predicted tension value, the system 101 can ensure that the most relevant and timely data is obtained for tension prediction and management. For example, if a high tension is predicted, the capture rate may be increased to allow for more frequent updates of the device data.

The display 150 may be provided to present information related to the operation and status of the elongate device 110. This information may include the predicted tension value, the categorized tension value, and any notifications or recommendations provided by the output interface 140. The display 150 allows for quick decision-making by medical professionals during the medical procedure.

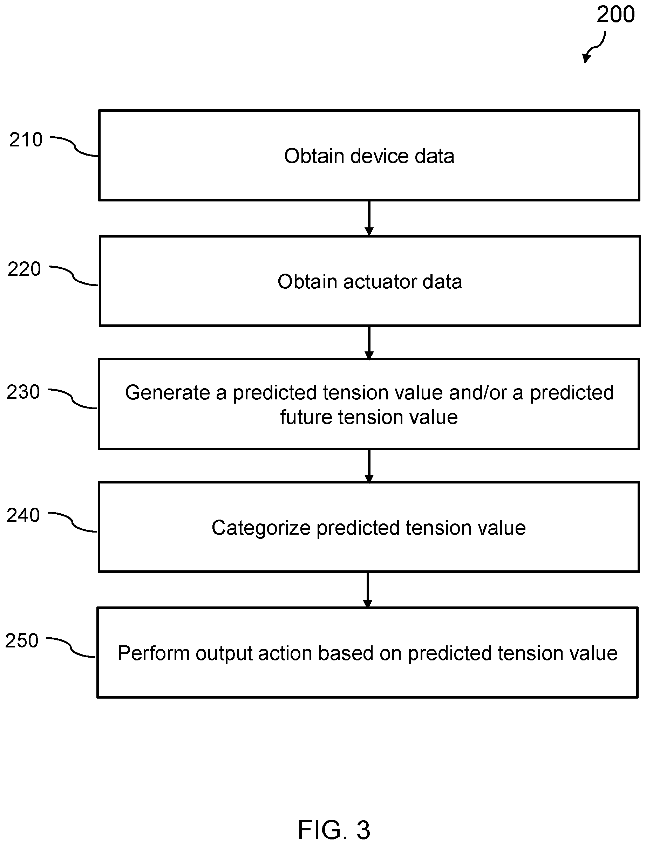

FIG. 3 shows a flowchart for a method 200 of monitoring tension in a medical instrument, according to an embodiment.

In step 210, device data is obtained, which indicates the shape and/or position of at least a portion of an elongate device. This device data may be obtained from various sources, such as medical imaging devices, sensors embedded in the elongate device, or other suitable sources. The device data provides valuable information about the current state of the elongate device, which can be used to predict the tension in the device.

In step 220, actuator data is obtained, which indicates the target shape and/or position of at least the portion of the elongate device. This actuator data may be based on user inputs, pre-programmed instructions, or other suitable sources. The actuator data provides information about the intended movement of the elongate device, which can be compared with the actual movement of the device as indicated by the device data.

It should be noted that steps 210 and 220 may be performed simultaneously, or in an alternative order. In any case, the device data and actuator data correspond to the respective actual and expected shape and/or position of the elongate device at the same point(s) in time.

In step 230, a predicted tension value and/or a predicted future tension value is generated. This prediction is based on processing the device data and actuator data using a tension prediction algorithm. The tension prediction algorithm may be a machine-learning based algorithm, which allows the system to continuously or periodically improve its prediction accuracy over time as more data to train with becomes available. The predicted tension value describes the degree of resistance against movement experienced by the elongate device. This prediction of tension allows for early detection of potentially dangerous situations, thereby enhancing patient safety during medical procedures.

In some cases, in step 240, the predicted tension value may be categorized. This categorization step may involve classifying the predicted tension value into one of several predefined categories or ranges. These categories or ranges may include at least one class associated with tension values in a safe range, and at least one class associated with tension values in a dangerous range. This categorization provides a clear and easily interpretable indication of the device's status, allowing for quick decision-making by medical professionals.

In step 250, an output action is performed based on the predicted tension value. This action could involve various responses such as controlling the actuator, providing notifications, or adjusting data capture parameters. For example, if a high/dangerous level of tension is predicted, the actuator may be controlled to adjust the shape and/or position of the elongate device to mitigate the tension. Alternatively, if a high/dangerous level of tension is predicted, the actuator may be controlled to cease movement of the elongate device. Notifications may be provided to the user indicating the predicted tension value, allowing the user to take appropriate action to manage the tension. The data capture parameters may be adjusted based on the predicted tension value to ensure that the most relevant and timely data is obtained for tension prediction and management.

In some aspects, the method may further include predicting if user inputs to the actuator will result in high tension, and providing a recommendation to the user indicating a recommended alternative input based on the predicted tension value. This recommendation can guide the user to adjust the control of the elongate device in a way that can reduce the tension. The recommendation may be based on the predicted tension value and other relevant factors, such as the characteristics of the elongate device and the current state of the medical procedure.

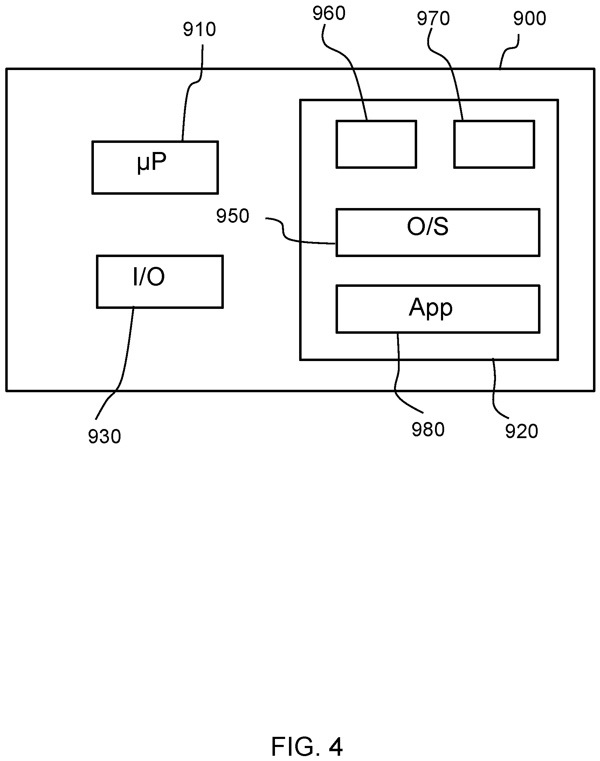

FIG. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a graphics processing unit (GPU), a tensor processing unit (TPU), a neural processing unit (NPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A system (101) for monitoring a medical instrument comprising an elongate device (110) and an actuator (112) configured to control movement of the elongate device, the system comprising:

   an input interface (120) configured to obtain device data indicating a shape and/or position of at least a portion of the elongate device, and to obtain actuator data indicating a target shape and/or position of at least the portion of the elongate device; and
   a processor (130) configured to process, with a tension prediction algorithm, the device data and the actuator data to generate a predicted tension value describing a degree of resistance against movement experienced by the elongate device.

2. The system of claim 1, wherein the processor (130) is further configured to categorize the predicted tension value into one of a plurality of classes, wherein the plurality of classes comprise at least one class associated with tension values in a safe range, and at least one class associated with tension values in a dangerous range.

3. The system of claim 1 or 2, wherein the tension prediction algorithm is a machine-learning based algorithm.

4. The system of claim 3, wherein the machine-learning algorithm is trained using a sequence of device data and corresponding actuator data, and associated tension values.

5. The system of any of claims 1-4, wherein the processor (130) is further configured to process, with the tension prediction algorithm, the device data and the actuator data to generate a predicted future tension value describing an expected change of the degree of resistance against movement experienced by the elongate device (110).

6. The system of any of claims 1-5, wherein the device data indicates a shape and/or position of the portion of the elongate device (110) over time, and the actuator data indicates the target shape and/or position of the portion of the elongate device over time.

7. The system of any of claims 1-6, wherein the device data comprises image data of at least the portion of the elongate device (110).

8. The system of claim 7, wherein the image data comprises a fluoroscopy image of at least the portion of the elongate device (110).

9. The system of any of claims 1-8, wherein the actuator data comprises input data describing one or more prompted actions of the actuator (112) to control movement of the elongate device (110).

10. The system of claim 1, wherein the predicted tension value is further based on one or more characteristics of the elongate device (110).

11. The system of any of claims 1-10, further comprising an output interface (140) configured, based on the predicted tension value, to:

control the actuator to adjust the shape and/or position of the elongate device;
control the actuator to cease movement of the elongate device;
provide a notification to the user indicating the predicted tension value;
provide a recommendation to the user indicating a recommended alternative input; and/or
control a capture rate at which the device data is obtained.

12. A medical device arrangement (100) comprising:

an elongate device (110);
an actuator (112) configured to control movement of the elongate device; and
the system (101) of any of claims 1-11.

13. The medical device arrangement of claim 12, wherein the actuator is controlled based, at least in part, on the predicted tension value.

14. A method for monitoring a medical instrument comprising an elongate device (110) and an actuator (112) configured to control movement of the elongate device, the method comprising:

obtaining (210) device data indicating a shape and/or position of at least a portion of the elongate device;
obtaining (220) actuator data indicating a target shape and/or position of at least the portion of the elongate device; and
processing, with a tension prediction algorithm, the device data and the actuator data to generate (230) a predicted tension value describing a degree of resistance against movement experienced by the elongate device.

15. A computer program comprising computer program code means adapted, when said computer program is run on a computer, to implement the method of claim 14.

FIG. 1

100

114

110 Elongate Device

Actuator

112

Monitoring
Unit

120 Input Interface

101

130 Processor

140 Output
interface

150 Display

FIG. 2

200

| 210 | Obtain device data |

| 220 | Obtain actuator data |

| 230 | Generate a predicted tension value and/or a predicted future tension value |

| 240 | Categorize predicted tension value |

| 250 | Perform output action based on predicted tension value |

FIG. 3

FIG. 4

# EUROPEAN SEARCH REPORT

Europäisches
Patentamt
European
Patent Office
Office européen
des brevets

**Application Number**

EP 24 21 5740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2024/268636 A1 (DUINDAM VINCENT [US] ET AL) 15 August 2024 (2024-08-15) * paragraphs [0005], [0027] - [0029], [0042] - [0051], [0056], [0070]; figures 1-8 * | 1,2,5-15 | INV.<br>A61B1/00<br>A61B1/005<br><br>ADD.<br>A61B34/20 |
| Y | US 2019/365201 A1 (NOONAN DAVID P [US] ET AL) 5 December 2019 (2019-12-05) * paragraphs [0035] - [0050], [0084] - [0104], [0123], [0124], [0130], [0131]; figures 1-2,8A-9L * | 1-4,6-15 | A61B34/30<br>A61B90/00 |
| Y | US 2011/319714 A1 (ROELLE MATTHEW J [US] ET AL) 29 December 2011 (2011-12-29) * paragraphs [0054], [0250] - [0258], [0297] - [0300]; figures 1A-2B, 64A,64B * | 1-4,7-15 | |
| Y | US 2023/165648 A1 (CAPLAN JAY [US] ET AL) 1 June 2023 (2023-06-01) * paragraphs [0124], [0136] - [0141], [0191] * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 2 May 2025 | Kink, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 4 751 634 A1**

## ANNEX TO THE EUROPEAN SEARCH REPORT
## ON EUROPEAN PATENT APPLICATION NO.

EP 24 21 5740

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2025

| Patent document cited in search report | Publication date | Patent family member(s) | Publication date |
|---|---|---|---|
| US 2024268636 A1 | 15-08-2024 | CN 109715037 A | 03-05-2019 |
| | | CN 115336961 A | 15-11-2022 |
| | | EP 3515281 A1 | 31-07-2019 |
| | | JP 7167035 B2 | 08-11-2022 |
| | | JP 2019529044 A | 17-10-2019 |
| | | KR 20190045380 A | 02-05-2019 |
| | | US 2019239723 A1 | 08-08-2019 |
| | | US 2024268636 A1 | 15-08-2024 |
| | | WO 2018057633 A1 | 29-03-2018 |
| US 2019365201 A1 | 05-12-2019 | US 2018177383 A1 | 28-06-2018 |
| | | US 2019365201 A1 | 05-12-2019 |
| US 2011319714 A1 | 29-12-2011 | US 2011319714 A1 | 29-12-2011 |
| | | US 2014357953 A1 | 04-12-2014 |
| | | US 2019125164 A1 | 02-05-2019 |
| | | US 2021353129 A1 | 18-11-2021 |
| | | US 2024099557 A1 | 28-03-2024 |
| US 2023165648 A1 | 01-06-2023 | EP 4090282 A1 | 23-11-2022 |
| | | IL 294671 A | 01-09-2022 |
| | | US 2023165648 A1 | 01-06-2023 |
| | | WO 2021146535 A1 | 22-07-2021 |

EPO FORM P0459

For more details about this annex : see Official Journal of the European Patent Office, No. 12/82

20